# EUROPÄISCHE PATENTSCHRIFT

(11) **EP 0 394 844 B1**
(45) Veröffentlichungstag und Bekanntmachung des Hinweises auf die Patenterteilung: **04.08.1993**
(21) Anmeldenummer: 90107440.1
(22) Anmeldetag: 19.04.1990
(51) Int. Cl.: C07C 69/716, C07C 67/313, C07C 59/185, C07C 51/373

(54) **Verfahren zur Herstellung von 4-Ketocarbonsäuren und deren Ester**
Process for the preparation of 4-ketocarboxylic acids and their esters
Procédé pour la préparation d'acides 4-cétocarboxyliques et de leurs esters

(30) Priorität: 22.04.1989 DE 3913333
(43) Veröffentlichungstag der Anmeldung: 31.10.1990
(73) Patentinhaber: BASF Aktiengesellschaft, 67063 Ludwigshafen (DE)
(72) Erfinder: Kaestner, Ralf, Dr., D-6700 Ludwigshafen (DE); Rieber, Norbert, Dr., D-6800 Mannheim 51 (DE)

(56) Entgegenhaltungen:
- DE-A- 1 618 821
- FR-A- 1 043 797
- US-A- 3 048 622

## Beschreibung

Die vorliegende Erfindung betrifft ein neues und verbessertes Verfahren zur Herstellung von 4-Ketocarbonsäuren und deren Ester aus 3,4-Epoxialkan- und/oder 4-Hydroxi-2-alkensäuren oder deren Ester in Gegenwart von geeigneten Katalysatoren.

Aus Journal of the American Oil Chemists Society, 42, 126 bis 129 (1965) ist bekannt, daß sich Ester langkettiger Carbonsäuren, die Epoxi-Gruppen enthalten, durch die Anwendung saurer Katalysatoren in die entsprechenden Keto-Säuren überführen lassen. Dabei wurden jedoch Mischungen zweier Keto-Säuren erhalten, bei denen die Keto-Gruppe an dem einen oder anderen der beiden zuvor durch ein Epoxi-Gruppe verbrückten Kohlenstoffatome zu finden war.

Ferner ist allgemein bekannt, daß 4-Hydroxi-alkansäureester sehr leicht Lactone bilden. Aus Tetrahedron Letters, 23, 4585 bis 4588 (1982) ist bekannt, daß z.B. der 4-Hydroxy-valeriansäure-methylester, der durch Reduktion des Lävulinsäuremethylesters mit Lithiumaluminiumhydrid hergestellt wurde, nicht isoliert werden konnte, ohne daß er zur Hälfte in das 5-Methyl-butyrolacton überging.

Der vorliegenden Erfindung lag daher die Aufgabe zugrunde, einen neuen und verbesserten Zugang zu den 4-Ketocarbonsäuren und deren Ester zu finden und den vorgenannten Nachteilen abzuhelfen.

Demgemäß wurde ein neues und verbessertes Verfahren zur Herstellung von 4-Ketocarbonsäuren und deren Ester der allgemeinen Formel I 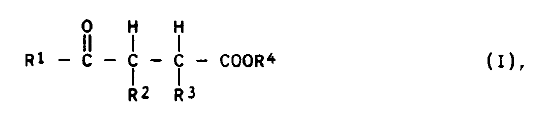
in der
- R¹, R², R³, R⁴: Wasserstoff, C₁-C₂₀-Alkyl, C₃-C₁₂-Cycloalkyl, C₄-C₂₀-Cycloalkylalkyl, C₄-C₂₀-Alkylcycloalkyl, C₅-C₃₀-Alkylcycloalkyl-alkyl oder C₇-C₂₀-Aralkyl bedeuten,

gefunden, welches dadurch gekennzeichnet ist, daß man 3,4-Epoxialkansäuren und deren Ester der allgemeinen Formel II 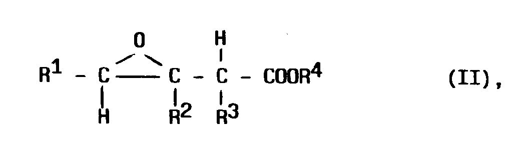
in der R¹ bis R⁴ die für die Verbindungen I genannten Bedeutungen haben, und/oder 4-Hydroxi-2-alkensäuren und deren Ester der allgemeinen Formel III 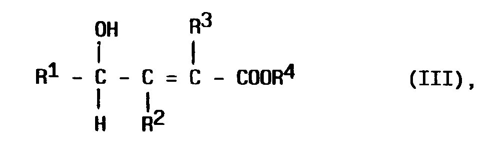
in der R¹ bis R⁴ die für die Verbindungen I genannten Bedeutungen haben, bei Temperaturen von 20 bis 600°C und Drücken von 0,01 bis 50 bar in Gegenwart von sauren oder basischen Katalysatoren isomerisiert.

Die 4-Ketocarbonsäuren und deren Ester I sind nach folgender Methode erhältlich:

Die Umsetzung erfolgt zwischen einer 3,4-Epoxialkan- und/oder einer 4-Hydroxi-2-alkensäure oder deren Ester in Gegenwart von geeigneten Katalysatoren bei Temperaturen von 20 bis 600°C und Drücken von 0,01 bis 50 bar nach folgender Reaktionsgleichung:

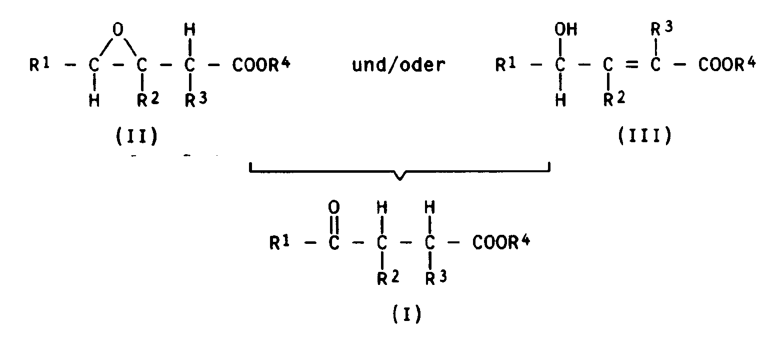

Die Isomerisierung kann diskontinuierlich oder kontinuierlich in der Gasphase oder vorzugsweise in der Flüssigphase durchgeführt werden.

Die Gasphasenreaktion kann beispielsweise bei Temperaturen von 100 bis 600°C und Drücken von 0,01 bis 50 bar, vorzugsweise bei 150 bis 500°C und Drücken von 0,1 bis 50 bar, besonders bevorzugt bei 200 bis 400°C und Drücken von 0,5 bis 5 bar durchgeführt werden. Bei der Umsetzung in der Gasphase hält man vorteilhaft eine Katalysatorbelastung von 0,1 bis 20, insbesondere von 0,5 bis 5 g Ausgangsstoff der Formel II je g Katalysator und Stunde ein (WHSV = g Einsatzgemisch/g Katalysator und Stunde). Die Gasphasenreaktion kann in einem Festbett oder in einem Wirbelbett ausgeführt werden.

Als Katalysatoren für eine Reaktion in der Gasphase eignen sich feste oxidische Katalysatoren. Das sind z.B. Oxide von Elementen der I. bis V. Hauptgruppe, der I. bis VIII. Nebengruppe des periodischen Systems der Elemente oder Oxide der Seltenen Erdmetalle oder Gemische der genannten Oxide. So sind beispielsweise Erdalkalioxide, wie Magnesiumoxid, Calciumoxid, Bariumoxid, weiterhin Bortrioxid, Aluminiumoxid, Siliciumdioxid, z.B. in Form von Kieselgel, Kieselgur oder Quarz, ferner Zinndioxid, Bismutoxid, Kupferoxid, Zinkoxid, Lanthanoxid, Titandioxid, Zirkondioxid, Vanadiumoxide, Chromoxide, Molybdänoxide, Wolframoxide, Manganoxide, Eisenoxide, Ceroxide, Neodymoxide oder Gemische derartiger Oxide geeignet. Die Katalysatoren können noch durch Aufbringen von Zusätzen, wie Säuren (z.B. Phosphorsäure) oder Basen (z.B. Natriumhydroxid) modifiziert werden. Bevorzugt sind Magnesiumoxid, Bortrioxid, Aluminiumoxid, Siliciumdioxid, Zinkoxid, Titanoxid, Zeolithe oder deren Gemische, von denen Aluminiumoxid-Katalysatoren ganz besonders geeignet sind.

Die bevorzugte Flüssigphasenreaktion kann beispielsweise als Suspensions-, Riesel- oder Sumpfreaktion bei Temperaturen von 20 bis 200°C und Drücken von 0,5 bis 20 bar, vorzugsweise bei Temperaturen von 50 bis 170°C und Drücken von 1 bis 5 bar, besonders bevorzugt bei Temperaturen von 60 bis 150°C und Atmosphärendruck durchgeführt werden. Geeignete Katalysatoren für diese Reaktion sind saure und basische Katalysatoren.

Als basische Katalysatoren verwendet man vorteilhaft Alkoholate von Alkali- oder Erdalkalimetallen, ferner von Aluminium oder Titan an. Weitere geeignete basische Katalysatoren sind Alkali- oder Erdalkaliamide oder -hydride. Geeignete Verbindungen sind beispielsweise Natriummethylat, Natriumethylat, Kaliumethylat, Magnesiumethylat, Aluminiumalkoholate oder Titanalkoholate, ferner Natriumamid, Kaliumamid, Natriumhydrid, Kaliumhydrid oder Calciumhydrid. In der Regel verwendet man Alkoholate, die sich von Alkoholen mit 1 bis 4 Kohlenstoffatomen ableiten. Weitere geeignete Katalysatoren sind basische Ionenaustauscher, z.B. vernetztes Polystyrol, das quarternäre Ammoniumgruppen enthält. Besonders bevorzugt werden Alkali- oder Erdalkalialkoholate oder basische Ionenaustauscher verwendet. Als basische Katalysatoren verwendet man weiterhin vorteilhaft cyclische, tertiäre Amine. Bevorzugte cyclische, tertiäre Amine sind
a) 5- bis 7-gliedrige, cyclische, tertiäre Amine, die 1 oder 2 Stickstoffatome und 1 Sauerstoffatom im Ring enthalten können, wobei mindestens 1 Stickstoffatom mit einem Alkylrest mit 1 bis 4 Kohlenstoffatomen substituiert ist. Geeignete Verbindungen sind beispielsweise N-Methyl-pyrrolidin, N-Methyl-piperidin, N-Methylhexamethylenimin, N-Ethylmorpholin oder N,N'-Dimethyl-piperazin. Besonders bevorzugt sind 5- bis 7-gliedrige, tertiäre, cyclische Amine mit nur 1 Stickstoffatom im Ring.
b) Aminopyridine 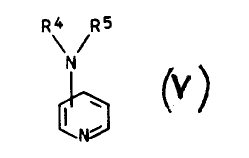 in der R⁴ und R⁵ gleich oder verschieden sind und jeweils einen Alkylrest mit 1 bis 4 Kohlenstoffatomen bezeichnen oder gemeinsam einen 5- bis 7-gliedrigen Ring bedeuten können, der zusätzlich ein Sauerstoff- oder Stickstoffatom enthalten kann. Besonders bevorzugt sind Verbindungen der Formel V, die sich vom 4-Amino-pyridin ableiten. Geeignete Verbindungen sind beispielsweise N,N-Dimethyl-4-aminopyridin, N,N-Diethyl-4-aminopyridin, 4-Morpholinopyridin oder 4-N-Piperazinopyridin.
c) N-substituierte Imidazole 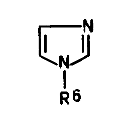 in der R⁶ einen Alkylrest mit 1 bis 18 Kohlenstoffatomen bezeichnet. Geeignete Verbindungen sind beispielsweise N-Methyl-, N-Ethyl-, N-Propyl- oder N-Dodecylimidazol,
d) Bicyclische Amidine, insbesondere 1,8-Diazabicyclo[5.4.0]undec-7-en oder 1,5-Diazabicyclo[4.3.0]non-5-en, oder
e) 1,4-Diazabicyclo[2.2.2]octan.

Es versteht sich, daß auch Gemische der vorgenannten cyclischen, tertiären Amine verwendet werden können.

Als saure Katalysatoren verwendet man vorteilhaft Halogenwasserstoffsäuren, Schwefelsäure, Salpetersäure, p-Toluolsulfonsäure oder Tetrafluoroborsäure. Weitere geeignete Katalysatoren sind saure Ionenaustauscher, z.B. vernetztes Polystyrol, das Sulfonsäuregruppen enthält.

Das Molverhältnis der 3,4-Epoxialkansäuren und deren Ester II und/oder der 4-Hydroxi-2-alkensäuren und deren Ester III zu den basischen oder sauren Katalysatoren beträgt vorteilhaft von 1000:1 bis 1:1, insbesondere von 200:1 bis 5:1.

Die Reaktionen in der Gasphase und in der Flüssigphase können gegebenenfalls in einem inerten Lösungsmittel wie Toluol, Tetrahydrofuran oder ein halogenierter Kohlenwasserstoff oder in einem C₁-C₁₈-Alkanol, einem Cycloalkanol mit 3 bis 7 Kohlenstoffatomen oder einem Aralkanol mt 7 bis 10 Kohlenstoffatomen durchgeführt werden. Dabei sollte der verwendete Alkohol demjenigen der Estergruppe des Produktes I entsprechen. Unter den Reaktionsbedingungen tritt bevorzugt Veresterung oder Umesterung ein, so daß es auf einfache Weise möglich ist, aus Estern einfacher Alkohole solche höherer Alkohole zu erhalten. Geeignete Alkohole sind beispielsweise Methanol, Ethanol, n-Propanol, Isopropanol, n-Butanol, sec.-Butanol, n-Pentanol, Decanol, Cyclohexanol, Cyclopentanol, Cycloheptanol oder Phenylethanol.

Es ist bei diesem Verfahren vorteilhaft, Mischungen der Verbindungen II und III, wie sie z.B. aus DE-A-39 13 330 bekannt sind, ohne vorherige Auftrennung direkt einzusetzen. Sie können jedoch auch einzeln eingesetzt werden, wenn sie nicht gemeinsam in einer Reaktion angefallen sind.

Die Umsetzung führt man bei diskontinuierlicher Arbeitsweise z.B. wie folgt durch: 4-Hydroxi-2-alkensäuren oder deren Ester, eventuell als Mischung mit 3,4-Epoxi-valeriansäureester wird mit einem Alkohol und den Katalysatoren auf die angegebene Reaktionstemperatur erhitzt und bei dieser Temperatur bis zum möglichst vollständigen Umsatz gerührt. Vorteilhaft wird der Katalysator, z.B. durch Filtration oder Neutralisation oder Extraktion mit Wasser, abgetrennt und das Reaktionsgemisch dann durch Destillation getrennt.

Die Substituenten R¹, R², R³ und R⁴ der 4-Keto-, der 3,4-Epoxialken- und der 4-Hydroxi-2-alkensäuren und deren Ester haben unabhängig voneinander folgende Bedeutungen:
- Wasserstoff,
- unverzweigtes oder verzweigtes C₁-C₂₀-Alkyl, bevorzugt C₁-C₈-Alkyl, besonders bevorzugt C₁-C₈-Alkyl wie Methyl, Ethyl, n-Propyl, iso-Propyl, n-Butyl, iso-Butyl, sec.-Butyl und tert.-Butyl,
- C₃-C₁₂-Cycloalkyl, bevorzugt C₃-C₈-Cycloalkyl wie Cyclopropyl, Cyclobutyl, Cyclopentyl, Cyclohexyl, Cycloheptyl und Cyclooctyl, besonders bevorzugt Cyclopentyl und Cyclohexyl,
- C₄-C₂₀-Cycloalkyl-alkyl, bevorzugt C₄-C₁₆-Cycloalkyl-alkyl, besonders bevorzugt C₄-C₁₂-Cycloalkyl-alkyl wie Cyclohexyl-methyl, 1-Cyclohexyl-ethyl und 2-Cyclohexyl-ethyl,
- C₄-C₂₀-Alkylcycloalkyl, bevorzugt C₄-C₁₆-Alkylcycloalkyl, besonders bevorzugt C₄-C₁₂-Alkylcycloalkyl wie 2-Methylcyclopentyl, 3-Methylcyclopentyl, 2-Methylcyclohexyl, 3-Methylcyclohexyl, 4-Methylcyclohexyl und 2,4-Dimethylcyclohexyl,
- C₅-C₃₀-Alkylcycloalkyl-alkyl, bevorzugt C₅-C₂₀-Alkylcycloalkyl-alkyl, besonders bevorzugt C₅-C₁₆-Alkylcycloalkyl-alkyl wie 2-Methylcyclopentyl-methyl und 2-Methylcyclohexyl-methyl,
- C₇-C₂₀-Aralkyl, bevorzugt C₇-C₁₄-Aralkyl, besonders bevorzugt C₇-C₁₀-Aralkyl wie Benzyl, 1-Phenethyl und 2-Phenethyl.

Der Substituent R¹ bedeutet vorzugsweise Wasserstoff oder C₁-C₄-Alkyl, R² vorzugsweise Wasserstoff oder Methyl, R³ vorzugsweise Wasserstoff oder Methyl und R⁴ vorzugsweise Wasserstoff oder C₁-C₁₆-Alkyl, besonders bevorzugt C₁-C₁₂-Alkyl wie Methyl, Ethyl, n-Propyl, iso-Propyl, n-Butyl, iso-Butyl, sec.-Butyl, tert.-Butyl, Pentyl, Hexyl, Heptyl, Octyl oder Dodecyl.

Bevorzugte 4-Ketocarbonsäuren und deren Ester I sind:
Lävulinsäure-methylester,
Lävulinsäure-ethylester.

Bevorzugte 3,4-Epoxi-alken-carbonsäuren und deren Ester I sind:
3,4-Epoxi-valeriansäure-methylester,
3,4-Epoxi-valeriansäure-ethylester,
3,4-Epoxi-valeriansäure-isopropylester,
3,4-Epoxi-valeriansäure-cyclohexylester,
3,4-Epoxi-valeriansäure-benzylester,
3,4-Epoxi-valeriansäuredodecylester.

Bevorzugte 4-Hydroxi-2-alken-carbonsäuren und deren Ester III sind:
4-Hydroxi-2-pentensäure-methylester,
4-Hydroxi-2-pentensäure-ethylester,
4-Hydroxi-2-pentensäure-isopropylester,
4-Hydroxi-2-pentensäure-cyclohexylester,
4-Hydroxi-2-pentensäure-benzylester,
4-Hydroxi-2-pentensäure-dodecylester.

Die nach dem Verfahren der Erfindung erhältlichen 4-Ketocarbonsäuren und deren Ester eignen sich z.B. als Weichmacher für Kunststoffe und sind wertvolle Zwischenprodukte bei der Herstellung von pharmazeutischen Wirkstoffen, z.B. des Antiphlogistikums Indometazin.

### Beispiele

### Beispiel 1

10 g trans-4-Hydroxi-2-pentensäure-methylester und 10 g 3,4-Epoxi-valeriansäure-methylester sowie 0,83 g Natriummethylat wurden in 200 ml trockenem Methanol gelöst und am Rückfluß erhitzt. Nach einer Stunde wurden weitere 0,42 g Natriummethylat zugegeben. Nach zwei Stunden wurde die erkaltete Mischung mit einer Lösung von Chlorwasserstoff in trockenem Methanol neutralisiert, das Lösungsmittel entfernt und der Rückstand durch Destillation gereinigt. Hierbei wurden 16,6 g Lävulinsäure-methylester vom Siedepunkt 84 bis 86°C/18 mbar erhalten (83 % d.Th.).

### Beispiel 2

20 g 4-Hydroxi-2-pentensäure-methylester wurden in 100 ml trockenem Methanol gelöst und 0,42 g Natriummethylat zugegeben. Die Lösung wurde in einem Autoklav eine Stunde lang auf 120°C. Nach dem Abkühlen wurden weitere 0,42 g Natriummethylat zugegeben und erneut eine Stunde auf 120°C erhitzt. Die erkaltete Reaktionsmischung wurde mit einer Lösung von Chlorwasserstoff in trockenem Methanol neutralisiert, das Lösungsmittel entfernt und der Rückstand in trockenem Methanol neutralisiert, das Lösungsmittel entfernt und der Rückstand durch Destillation gereinigt. Hierbei wurden 13,4 g Lävulinsäure-methylester vom Siedepunkt 84 bis 86°C/18 mbar erhalten (67 % d.Th.).

### Beispiel 3

20 g 3,4-Epoxi-valeriansäure-methylester wurden in 100 ml trockenem Methanol gelöst und am Rückfluß erhitzt. Dabei wurde der pH-Wert durch Zutropfen von einer 0,3 N Lösung von Natriummethylat in trockenem Methanol zwischen 10,5 und 10,8 gehalten. Nach 5 Stunden ließ man abkühlen und neutralisierte mit einer Lösung von Chlorwasserstoff in trockenem Methanol. Das Lösungsmittel wurde entfernt und der Rückstand durch Destillation gereinigt. Hierbei wurden 12,8 g Lävulinsäure-methylester vom Siedepunkt 84 bis 86°C/18 mbar erhalten (63,5 % d.Th.).

### Beispiel 4

20 g 3,4-Epoxi-valeriansäure-methylester und 1,25 g einer 54 %gen Lösung von Tetrafluoroborsäure in Diethylether wurden in 200 ml Toluol gelöst. Die Reaktionsmischung wurde eine Stunde lang am Rückfluß erwärmt. Nach dem Abkühlen wurde die Reaktionsmischung mit Triethylamin neutralisiert, das Lösungsmittel entfernt und der Rückstand durch Destillation gereinigt. Hierbei wurden 6,2 g Lävulinsäure-methylester vom Siedepunkt 84 bis 86°C/18 mbar erhalten (31 % d.Th.).

### Beispiel 5

Ein zylindrischer Ofen mit 3 cm Innendurchmesser und 30 cm Höhe wurde mit pelletiertem Aluminiumoxid gefüllt und auf 300°C erhitzt. Bei einem Druck von 1 mbar wurden 150 g 4-Hydroxi-2-pentensäure-methylester verdampft. Der Dampf wurde in einem Stickstoffstrom durch das erhitzte Aluminiumoxid geleitet und danach in einem Produktkühler kondensiert. Es wurden 133 g Kondensat gewonnen, das nach gaschromatographischer Analyse zu 80 % aus Lävulinsäure-methylester bestand, der in über 95 %iger Reinheit isoliert werden konnte.

### Beispiel 6

11,5 g 4-Hydroxi-2-pentensäure-methylester, 1,22 g 4-Dimethylaminopyridin und 100 ml Methanol werden in einem Glasuatoklaven 7 Stunden auf 120°C erhitzt. Nach gaschromatographischer Analyse besteht das Reaktionsgemisch außer aus Methanol und 4-Dimethylaminopyridin zu 93 % aus Lävulinsäure-methylester, zu 2 % aus nicht umgesetztem Ausgangsprodukt und ca. 5 % höhersiedenden Nebenprodukten.

### Beispiel 7

100 g des stark basischen Ionenaustauschers Amberlyst A26 wurden in einer Chromatographiesäule mit 2 l einer 1 N Lösung von Natriummethylat in Methanol und danach mit 1 l trockenem Methanol gewaschen. 10 g 4-Hydroxi-2-pentensäuremethylester wurden in 100 ml trockenem Methanol gelöst und 15 g des Ionenaustauschers darin suspendiert. Es wurde 3 Stunden am Rückfluß erhitzt, dann wurden weitere 2 g des Ionenaustauschers zugegeben und noch einmal 2 Stunden am Rückfluß erhitzt. Die Suspension wurde filtriert. Die gaschromatographische Analyse zeigte, daß das Filtrat außer aus dem Lösungsmittel zu 98 % aus Lävulinsäuremethylester bestand.

### Beispiel 8

20 g 4-Hydroxi-2-pentensäure-methylester wurden in 120 ml Cyclohexanol gelöst und 1,08 g Natriumhydrid zugegeben. Die Lösung wurde 24 Stunden am Rückfluß erhitzt. Nach dem Abkühlen wurde mit einer Lösung von Chlorwasserstoff in Methanol neutralisiert und danach filtriert. Aus dem Filtrat wurden durch Destillation 7,2 g Lävulinsäure-cyclohexylester vom Siedepunkt 116 bis 118°C/1 mbar gewonnen.

### Beispiel 9

20 g 4-Hydroxi-2-pentensäure-methylester wurden in 120 ml Hexanol gelöst und 1,2 g Natriummethylat zugegeben. Die Suspension wurde 24 Stunden bei 120°C gerührt. Danach wurde filtriert, und aus dem Filtrat wurden durch Destillation 5,8 g Lävulinsäure-n-hexylester vom Siedepunkt 132°C/12 mbar gewonnen.

## Patentansprüche

1. Verfahren zur Herstellung von 4-Ketocarbonsäuren und deren Ester der allgemeinen Formel I 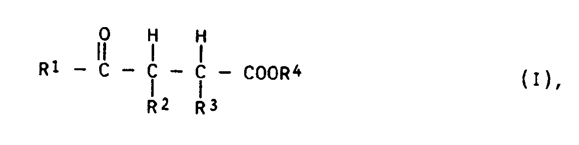 in der
R¹, R², R³, R⁴ Wasserstoff, C₁-C₂₀-Alkyl, C₃-C₁₂-Cycloalkyl, C₄-C₂₀-Cycloalkyl-alkyl, C₄-C₂₀-Alkylcycloalkyl, C₅-C₃₀-Alkylcycloalkyl-alkyl oder C₇-C₂₀-Aralkyl bedeuten,
dadurch gekennzeichnet, daß man 3,4-Epoxialkansäuren und deren Ester der allgemeinen Formel II 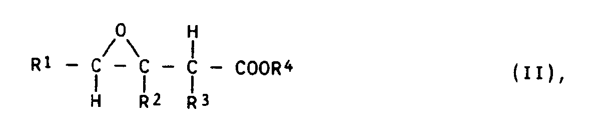 in der R¹ bis R⁴ die für die Verbindungen I genannten Bedeutungen haben, und/oder 4-Hydroxi-2-alkensäuren und deren Ester der allgemeinen Formel III 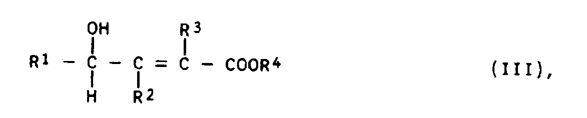 in der R¹ bis R⁴ die für die Verbindungen I genannten Bedeutungen haben, bei Temperaturen von 20 bis 600°C und Drücken von 0,01 bis 50 bar in Gegenwart von sauren oder basischen Katalysatoren isomerisiert.

2. Verfahren nach Anspruch 1, dadurch gekennzeichnet, daß man als basische Katalysatoren Alkoholate, Amide oder Hydride von Alkali- oder Erdalkalimetallen oder basischen Ionenaustauschern einsetzt.

3. Verfahren nach Anspruch 1, dadurch gekennzeichnet, daß man als basische Katalysatoren cyclische tertiäre Amine einsetzt.

4. Verfahren nach Anspruch 1, dadurch gekennzeichnet, daß man als saure Katalysatoren Halogenwasserstoffsäure, Schwefelsäure, Salpetersäure, Tetrafluoroborsäure oder saure Ionenaustauscher einsetzt.

5. Verfahren nach Anspruch 1, dadurch gekennzeichnet, daß man die Umsetzung in der Gasphase an sauren oder basischen Oxiden von Elementen der I. bis V. Hauptgruppe, der I. bis VIII. Nebengruppe des Periodensystems der Elemente oder Oxiden der seltenen Erdmetallen durchführt.

6. Verfahren nach Anspruch 1, dadurch gekennzeichnet, daß man als saure oder basische Katalysatoren Magnesiumoxid, Aluminiumoxid, Siliciumoxid, Titandioxid oder Zeolithen einsetzt.

7. Verfahren nach Anspruch 1, dadurch gekennzeichnet, daß man die Umsetzung in Gegenwart von Aluminiumoxid durchführt.

## Claims

1. A process for preparing 4-keto carboxylic acids and their esters of the formula I 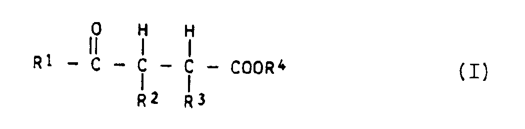 where
R¹, R², R³ and R⁴ are each hydrogen, C₁-C₂₀-alkyl, C₃-C₁₂-cycloalkyl, C₄-C₂₀-cycloalkylalkyl, C₄-C₂₀-alkylcycloalkyl, C₅-C₃₀-alkylcycloalkylalkyl or C₇-C₂₀-aralkyl,
which comprises isomerizing 3, 4-epoxyalkanoic acids and their esters of the formula II 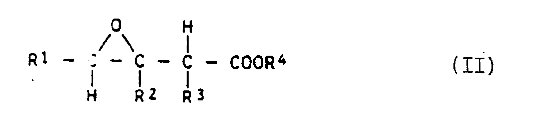 where R¹ to R⁴ have the meanings specified for the compounds 1, and/or 4-hydroxy-2-alkenoic acids and their esters of the formula III 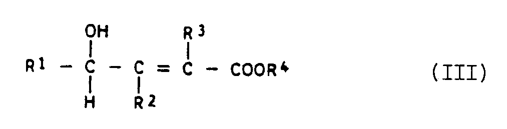 where R¹ to R⁴ have the meanings specified for the compounds I, at from 20 to 600°C and under from 0.01 to 50 bar in the presence of acidic or basic catalysts.

2. A process as claimed in claim 1, wherein alcoholates, amides or hydrides of alkali metals or alkaline earth metals or basic ion exchangers are employed as basic catalysts.

3. A process as claimed in claim 1, wherein cyclic tertiary amines are employed as basic catalysts.

4. A process as claimed in claim 1, wherein hydrohalic acid, sulfuric acid, nitric acid, tetrafluoroboric acid or acid ion exchangers are employed as acid catalysts.

5. A process as claimed in claim 1, wherein the reaction is carried out in the gas phase on acidic or basic oxides of elements of group Ia to Va, of group Ib to VIII of the periodic table of the elements or oxides of the rare earth metals.

6. A process as claimed in claim 1, wherein magnesium oxide, aluminum oxide, silicon oxide, titanium dioxide or zeolites are employed as acid or basic catalysts.

7. A process as claimed in claim 1, wherein the reaction is carried out in the presence of aluminum oxide.

## Revendications

1. Procédé de préparation d'acides 4-cétocarboxyliques et de leurs esters de formule générale 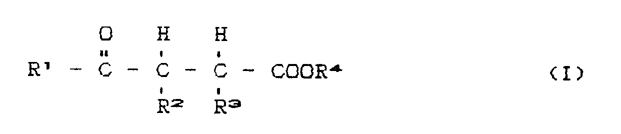 dans laquelle
R¹, R², R³, R⁴ représentent des atomes d'hydrogène ou des restes alkyle en C₁-C₂₀, cycloalkyle en C₃-C₁₂, cycloalkyle en C₄-C₂₀, alkylcycloakyle en C⁴-C₂₀, alkylcycloalkylalkyle en C₅-C₃₀ ou aralkyle en C₇-C₂₀,
caractérisé en ce qu'on isomérisé des acides 3,4-époxy-alcanoïques et leurs esters de formule générale II 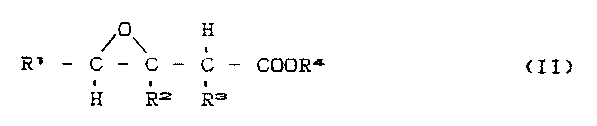 dans laquelle R¹ à R⁴ ont les significations indiquées à propos des composés I, et/ou des acides 4-hydroxy-2-alcanoïques et leurs esters de formule générale III 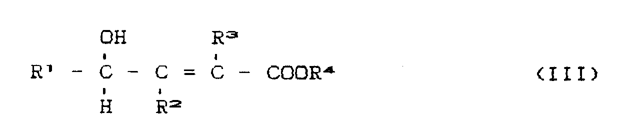 dans laquelle R¹ à R⁴ on les significations indiquées à propos des composés I, à des températures de 20 à 600°C et sous des pressions de 0,01 à 50 bar, en présence de catalyseurs acides ou basiques.

2. Procédé selon la revendication 1, caractérisé en ce qu'on utilise, comme catalyseurs basiques, des alcoolates, des amidures ou des hydrures de métaux alcalins ou alcalino-terreux ou des échangeurs d'ions basiques.

3. Procédé selon la revendication 1, caractérisé en ce qu'on utilise, comme catalyseurs basiques, des amines tertiaires cycliques.

4. Procédé selon la revendication 1, caractérisé en ce qu'on utilise, comme catalyseurs acides, des hydracides halogénés, de l'acide sulfurique, de l'acide nitrique, de l'acide tétrafluoroborique ou des échangeurs d'ions acides.

5. Procédé selon la revendication 1, caractérisé en ce qu'on conduit la réaction en phase gazeuse en présence d'oxydes acides ou basiques d'éléments des groupes IA à VA ou des groupes IB à VII et VIII du système périodique des éléments ou en présence d'oxydes des métaux des terres rares.

6. Procédé selon la revendication 1, caractérisé en ce qu'on utilise, comme catalyseurs acides ou basiques, de l'oxyde de magnésium, de l'oxyde d'aluminium, de l'oxyde de silicium, du dioxyde de titane ou des zéolithes.

7. Procédé selon la revendication 1, caractérisé en ce qu'on conduit la réaction en présence d'oxyde d'aluminium.
